# EUROPEAN PATENT APPLICATION

(11) **EP 4 169 503 A1**
(43) Date of publication of application: **26.04.2023**
(21) Application number: 21203645.3
(22) Date of filing: 20.10.2021
(51) Int. Cl.: A61K 9/00, A61K 9/48, A61K 9/70

(54) **COMPOSITION COMPRISING GELATIN, GELATIN FILMS AND CAPSULES**

(71) Applicant: Tessenderlo Group NV, 1050 Brussels (BE)
(72) Inventor: Maertens, Faye, 1050 Brussel (BE); MANDOLESI, Carlos, 1050 Brussels (BE)
(74) Representative: Hoyng Rokh Monegier B.V.

(57) **Abstract**

The present invention provides a film comprising gelatin as film former and an effective amount of surfactant to provide a retarded dissolution rate at low pH, in comparison to the dissolution rate at neutral pH. In particular, the effective amount of surfactant is such to provide a dissolution rate such that 50% or less of a film is dissolved within 120 minutes at 37° C at a pH of about 1, while more than 50% of the film is dissolved in 45 min or less at pH 6.8 at 37°C. The surfactants include anionic surfactants, cationic surfactants, non-ionic surfactants and amphoteric surfactants. A preferred film comprises gelatin and about 8 wt.% or more on dry weight basis of an anionic surfactant having a C₈-C₂₄ alkyl chain.

## Description

### Field of the invention

The invention relates to a solid homogeneous composition comprising gelatin and a surfactant, more particularly to a film comprising gelatin as a film former and a surfactant, and capsules comprising such film. The invention also relates to a method for preparing the film, and to a use of a surfactant in gelatin compositions, films and capsules.

The composition, films and capsules of the invention show a slow dissolution rate at low pH, and a fast dissolution rate at neutral pH. Therefore, capsules of the present invention with active ingredients provide a delayed release profile at low pH, and a fast release profile at neutral pH.

### Background of the invention

Gelatin is a translucent, tasteless, water-soluble protein derived from boiling animal tissues. It is commonly used as an excipient for pharmaceutical and food supplement applications, and is used for making for example hard or soft gelatin capsules.

Hard capsules are generally filled with solid products such as powders, pellets, granules or mini tablets, for examples solids that have a bad taste or would be irritating upon direct contact with the gastrointestinal tract. Hard capsules are comprised of two halves - a capsule body and a capsule cap - that fit together.

Soft capsules (also called soft gel) generally are useful for drug formulations containing liquids, oils, pastes or suspensions. Advantages of soft capsules are product stability (prevention of oxidative degradation); easiness to swallow, and good dose uniformity for low-dose drugs (as liquid flow and homogeneity is more precise than powder flow).

The melting point of gelatin is near body temperature, and gelatin dissolves more easily in an acidic environment than in a neutral environment. Therefore, gelatin capsules are easily digested in the stomach, thus also releasing their content in the stomach. For drugs or food supplements that are sensitive to the acid conditions of the stomach (e.g. enzymes, peptides or proton pump inhibitors), that would be irritating for the gastric mucosa (e.g. non-steroidal anti-inflammatory drugs, NSAIDs), and/or that need to be delivered to the intestine (e.g. colon) to obtain a local effect, it is preferred that the capsules reach the intestines before releasing their contents. It may also be preferred to have the capsules reach the intestines before releasing their contents, to minimize burps and acid reflux.

To achieve such a specific dissolution profile several solutions exist, such as delayed release capsules, and in particular so-called 'enteric capsules'. Delayed release capsules are designed to release their content some time after administration, generally in a gradual fashion, which can be advantageous. In some instances, after this period has elapsed, the release may be rapid. Enteric capsules are a subgroup of delayed release capsules. Enteric capsules release very little to none of their content at low pH (such as in the stomach), but release the majority of their content at higher pH (such as in the intestines).

In order to achieve such enteric behavior with gelatin capsules, such capsules generally are coated with a so-called enteric coating. This coating is a polymer barrier that protects the gelatin capsule from premature disintegration at low pH. The coating is typically pH activated, and remains intact at low pH but dissolves readily as the pH of the intestines increases.

More specifically, enteric behavior is obtained if the enteric dosage form does not dissolve or disintegrate in gastric acidity for a specified amount of time (usually two hours in 0.1 M hydrochloric acid at 37 °C). Further, the enteric dosage forms must release their contents in simulated intestinal environments (e.g., in buffers of pH values at about 6.8 within certain time periods). Detailed evaluation techniques are described in national and international pharmacopoeia, like the European Pharmacopoeia.

Examples of enteric coatings for e.g. capsules are described in CN103800303A, CN104434870A, US5330759A, EP3090637A and EP2255794A.

Providing such protective layer requires an extra and rather complicated process step in the production process of the capsules. This can result in longer processing times, a more complex process, and yield loss. Moreover, the added process step can result in sealing issues.

Further, numerous process parameters can impact the coating process and the quality of the coating. This can result in unwanted variability in the release behavior due to variability in coating and defects such as chipping, cracks and peeling. Additionally, while aqueous solvents used during the coating process may have an impact on drying time and microbiological stability, organic solvents may pose safety and environmental hazards and solvent residue issues. Some acid resistant coatings like Eudragit may not be suitable for direct coating of gelatin capsules due to insufficient film adhesion and a brittleness of formed films. The opacity of the capsules can be affected by the coating, resulting in less attractive capsules.

When coating soft gelatin capsules, additional difficulties may arise due to their smooth, non-absorptive surfaces, flexibility and elasticity.

It is an object of the present invention to provide a film or capsule comprising the film which exhibits a slow dissolution rate at low pH and a fast dissolution rate at neutral pH.

It is a further object of the invention, to provide capsules having a desired enteric release profile, without requiring a coating.

It is further an object of the invention to provide a more efficient method for preparing a film which exhibits a relatively slow dissolution rate at low pH, while showing a fast dissolution rate at neutral pH.

### Summary of the invention

One or more of above objects are achieved according to the present invention, that provides an homogeneous solid composition comprising gelatin and a surfactant, in particular a film comprising gelatin as film former and a surfactant, preferably an effective amount of a surfactant, to provide a slow dissolution rate at low pH in aqueous medium, in comparison to a fast dissolution rate at neutral pH in aqueous medium.

Preferably the dissolution rate is such that
- 50% or less of a film is dissolved within 120 minutes at 37° C at a pH of about 1, more particular in 0.1 M HCI in water, and
- 50% or more of the film is dissolved within 45 minutes or less at pH 6.8 at 37°C, more particular in a 0.05 M sodium phosphate buffer.

The dissolution rates of the film preferably are such as to provide a capsule with a delayed release in the stomach, more preferably a capsule with an enteric behavior (less than 10% release in the stomach in 2 hours, while providing full release in the intestines).

Depending on the surfactant, the necessary or useful amount may vary, but generally the amount of surfactant is about 3 wt.% or more, preferably about 5 wt.% or more, and may be 6 wt.% or more, 7 wt. % or more, 8 wt.% or more, 9 wt. % or more, 10 wt. % or more. The skilled person can easily assess the required amount for a suitable delayed dissolution at low pH.

Generally, the amount of surfactant is about 30 wt% or lower, preferably about 20 wt% or less, and even more preferred, about 15 wt% or less.

The weight percentages are defined - unless otherwise specified - in this application as relative to a dried gelatin film (or capsule shell) with 12 wt% moisture.

Unexpectedly, incorporating an effective amount of a surfactant in the film results in a delayed dissolution rate at low pH, yet results in a rapid dissolution rate at neutral pH. The capsule comprising the film thus shows the desired delayed release behavior.

Suitable surfactants include anionic surfactants, cationic surfactants, non-ionic surfactants and amphoteric surfactants.

In one preferred embodiment, the surfactant is an anionic surfactant.

In another preferred embodiment, the surfactant is a cationic surfactant.

In another preferred embodiment, the surfactant is a non-ionic surfactant.

In another preferred embodiment, the surfactant is an amphoteric surfactant.

There is further provided a capsule wherein the film of the invention encapsulates filling material, wherein such filling material preferably comprises a food supplement or an active pharmaceutical ingredient.

There is further provided a method of preparing a film comprising the steps of:
(a) preparing an aqueous gelatin solution comprising 20 wt.% or more and 50 wt.% or less of gelatin and 3 wt.% or more of a surfactant; and
(b) forming a film by drying,
wherein the film exhibits a slow dissolution rate at pH 1, and a fast dissolution rate at neutral pH.

The method of the invention does not require an additional coating step with a polymer to provide enteric behavior, resulting in a more efficient process.

In a particular preferred embodiment, the present invention provides a film comprising gelatin and about 8 wt.% or more of an anionic surfactant having a C₈-C₂₄ alkyl chain.

There is further provided a use of a surfactant in gelatin film, preferably an anionic surfactant having a C₈-C₂₄ alkyl chain, preferably in an amount of about 8 wt.% or more, to achieve a slow dissolution rate at low pH while having fast dissolution rate at neutral pH.

EP1210936A discloses a process for the preparation of gelatin capsules, which has reduced incidence of static electricity and enhanced capsule film surface lubrication while using 1 wt.% or less of surfactant. The capsules are not disclosed to show an enteric or delayed dissolution profile in acidic environment.

### Detailed description

The film of the present invention comprises gelatin as film former and an effective amount of surfactant to provide a delayed dissolution rate of the gelatin film at low pH, in comparison to a fast dissolution rate at neutral pH.

### Gelatin

Preferably, the film comprises gelatin in an amount of about 30 wt.% or higher, and about 87 wt.% or lower, preferably about 35 wt.% or higher, and about 80 wt.% or lower on dry weight basis.

The amounts are given in weight, relative to the film having 12 wt% moisture content unless otherwise specified.

The gelatin may comprise type B gelatin (alkali processed), or type A gelatin (acid treated).

The gelatin may be derived from any suitable raw material such as bones and/or skins/hides and/or cartilage of various animals, such as pig, beef, poultry, goat, donkey and the like or from scales and/or skin of fish. Preferably the gelatin is derived from bones and/or hides of pig and/or beef.

In a preferred embodiment, the gelatin is preferably of the "high Bloom" type, more preferably having a Bloom strength of about 200 to about 280 g Bloom. Preferably, the viscosity is about 4 to about 5 mPa s.

The Bloom strength is measured according to the Bloom test as commonly known and described in the GME monograph (version 15, of October 2020). The viscosity is also determined according to this GME monograph: For a 6.67 % solution of gelatin, the flow time of 100 ml through a standard pipette is measured at 60 °C, and the dynamic viscosity calculated.

The wt% values when referring to dry basis, given in this description are measured at 12 wt% moisture.

The high Bloom type is preferably used in hard capsules. The gelatin type for hard capsules often is of the B-type, although A-type hard capsules exist.

In another preferred embodiment, the gelatin is preferably of the "medium Bloom" type, more preferably about 120 to about 220 g Bloom. Preferably, the viscosity is about 2 to about 4 mPa s.

The medium Bloom type preferably is used in soft gel capsules. Soft capsules are more often of an A type gelatin, but soft capsules of the B type gelatin are also common.

### Surfactant

In one preferred embodiment, the surfactant is an anionic surfactant. Suitable anionic surfactants include sodium lauryl sulfate (SLS; sodium dodecyl sulfate), sodium lauryl ether sulfate, ammonium lauryl sulfate, ammonium lauryl ether sulfate, sodium stearate, magnesium stearate, calcium stearate, sodium stearyl fumarate, calcium stearyl fumarate, sodium stearoyl-2-lactylate, calcium stearoyl-2-lactylate, dioctyl sodium sulfosuccinate, sodium cetearyl sulfate.

In a further preferred embodiment, the surfactant is a cationic surfactant. Suitable cationic surfactants include quaternary ammonium compounds such as cetrimonium bromide, cetylpyridinium chloride and the like.

In a further preferred embodiment, the surfactant is a non-ionic surfactant. Suitable non-ionic surfactants include polyoxyethylene alkyl ethers (macrogols) such as macrogol cetostearyl ether, macrogol lauryl ether, macrogol oleyl ether, macrogol stearyl ether and the like; sorbitan esters such as sorbitan monostearate (E491), sorbitan tristearate (E492), sorbitan monolaurate (E493), sorbitan monooleate (E494), sorbitan monopalmitate (E495), sorbitan trioleate (E496) and the like; polyoxyethylene sorbitan fatty acid esters (polysorbates) such as polysorbate 20 (E432), polysorbate 40 (E434), polysorbate 60 (E435), polysorbate 65 (E436), polysorbate 80 (E433), polyoxyl 8 stearate (E430), polyoxyl 40 stearate (E431) and the like; polyoxyethylene castor oil derivatives such as sucrose esters such as partial polyglycerol esters of polycondensed fatty acids of castor oil (E498); sucrose esters of fatty acids (E473); glucose esters and derivatives thereof; glycerol esters such as glyceryl monooleate and the like; ethoxylated alcohols such as ethoxylated alkylphenols, ethoxylated fatty amines, ethoxylated fatty acids, ethoxylated fatty esters and oils, fatty acids, monoglycerides and derivatives, propoxylated and ethoxylated fatty acids, block copolymers of ethylene oxide (EO) and propylene oxide (PO), silicone-based surfactants and mixtures of organosilicon surfactant with non-ionic or ionic surfactants; polysaccharides, copolymers of acrylamide and acrylic acid; and acetylenic diol derivatives or tristyrylphenols, ethoxylated sorbitan esters.

In a further preferred embodiment, the surfactant is an amphoteric surfactant. Suitable amphoteric surfactants include lecithin (E322) and lecithin derivatives; and imidazolines and imidazoline derivatives; triethanolamine oleate; betaines such as cocamidopropyl betaine and the like.

A particular preferred film of the invention comprises gelatin, and at least one anionic surfactant having a C₈-C₂₄ alkyl chain in an amount of about 8 wt.% or more, preferably 10 wt% or more.

A particularly preferred anionic surfactant has a C₈-C₂₄ alkyl chain (long chain alkyl group) and an anionic group (hereinafter also abbreviated as long chain anionic surfactant).

Preferably, the anionic group of this long chain anionic surfactant is a sulfate group, sulfonic group, phosphate group, phosphonate group, or carboxylate group. More preferably, the anionic group is a strong acid group like a sulfate group or sulfonic acid group.

The long chain alkyl group can be linear or branched.

Preferably, the long chain anionic surfactant comprises ether or ester groups.

The molecular weight of the long chain anionic surfactant is preferably about 250 g/mol to about 600 g/mol, more preferably about 300 g/mol to about 400 g/mol.

Preferably, the long chain anionic surfactant is chosen from the group consisting of sodium lauryl sulfate (SLS), sodium lauryl ether sulfate, ammonium lauryl sulfate, ammonium lauryl ether sulfate, sodium stearate, magnesium stearate, calcium stearate, sodium stearyl fumarate, calcium stearyl fumarate, sodium stearoyl-2-lactylate, calcium stearoyl-2-lactylate, dioctyl sodium sulfosuccinate, sodium cetearyl sulfate.

More preferably, the long chain anionic surfactant is chosen from the group consisting of sodium lauryl sulfate (SLS) and sodium lauryl ether sulfate; and even more preferably is sodium lauryl sulfate (SLS).

Sodium lauryl sulfate is widely used in cosmetics and nonparenteral pharmaceutical formulations, i.e. oral and topical applications. It is GRAS listed and included in the FDA Inactive Ingredients Guide (dental preparations; oral capsules, suspensions, and tablets; topical and vaginal preparations). It is included in nonparenteral medicines licensed in the UK, and in the Canadian List of Acceptable Non-medicinal Ingredients.

Sodium cetyl sulfate and sodium stearyl sulfate, like sodium lauryl sulfate, are fatty acid sulfates. Sodium cetearyl sulfate is a mixture of sodium cetyl sulfate and sodium stearyl sulfate. They are known pharmaceutical excipients.

Sodium lauryl ether sulfate (SLES), also known as sodium laureth sulfate, is a fatty acid sulfate, comprising ethoxyl groups in the fatty acid chain. It has the following formula:

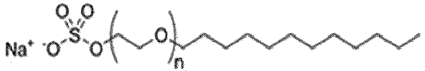

Ammonium lauryl ether sulfate has ammonium as the cation instead of sodium. Both sodium lauryl ether sulfate and ammonium lauryl ether sulfate are known pharmaceutical excipients.

Sodium, potassium, and calcium salts of fatty acids are approved as food additives in the EU under E-number E470a, and magnesium salts of fatty acids are approved under E-number E470b. These include salts of stearic acid, lauric acid, and the like.

Dioctyl sodium sulfosuccinate, also known as docusate sodium and having E-number E480, is a known pharmaceutical excipient. Docusate sodium has the following formula:

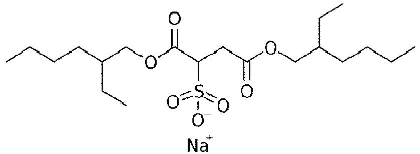

Sodium stearoyl-2-lactylate and calcium stearoyl-2-lactylate are approved as food additives in the EU under E-numbers E481 and E482 respectively, and have the following formulas:

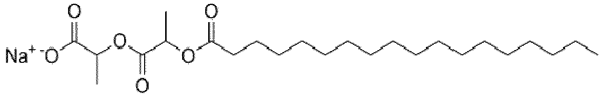

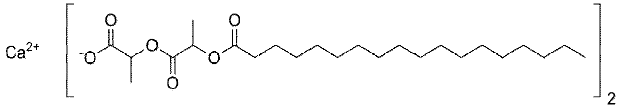

Sodium stearyl fumarate and calcium stearyl fumarate are known under E-numbers E485 and E486 respectively, and have the following formulas:

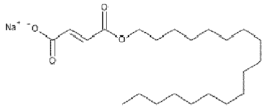

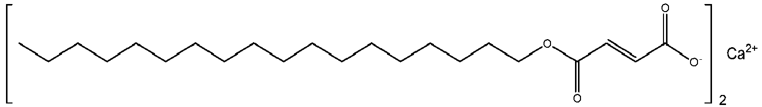

### Plasticizer and other components

Preferably the film - if a soft gel is aimed for - comprises a plasticizer. Plasticizers can be used to modify the physical properties of the film, and to make soft capsule gelatin films elastic and pliable.

Preferably, the soft gel film comprises the plasticizer in an amount of about 10 wt.% to about 40 wt.%, more preferably between about 15 wt% to about 30 wt%.

Preferably, the plasticizer is selected from the group consisting of glycerol, propylene glycol, polyethylene glycol, sugar alcohols with 3 to 6 carbon atoms (such as sorbitol and mannitol), organic esters such as triethyl citrate and diethyl phthalate, and combinations thereof. More preferably, said plasticizer is selected from the group consisting of glycerol, sorbitol, polyethylene glycol and combinations thereof. Even more preferably, said plasticizer comprises glycerol.

In a further preferred embodiment, the film further comprises an acid, preferably a weak acid, like for example citric acid, lactic acid, glycolic acid, or hydrochloric acid. More preferably, the film comprises citric acid or lactic acid.

The film may further comprise colorants, like synthetic dyes, such as azo dyes, or non-azo dyes, or natural dyes. The film may further comprise an opacifier, such as TiO₂. This can allow to produce an opaque shell to prevent photodegradation of light-sensitive filling material.

### Film and capsules

The film and the capsule comprising the film show a retarded (relatively slow) dissolution rate at low pH, and a fast dissolution rate at neutral pH. Thereby, capsules comprising an active compound show a delayed release at low pH but dissolve rapidly at neutral pH, and thereby provide full release of the active compound at such neutral pH.

Preferably, 50% or less, more preferably 40% or less, even more preferably 30% or less, yet even more preferably 20% or less of the film is dissolved within 120 minutes at 37° C and at a pH of about 1. For example, a 0.1 M hydrochloric acid solution can be used to perform this test.

Preferably, 50% or more, more preferably 70% or more, even more preferably 80% or more, yet even more preferably 90% or more of the film is dissolved within 45 minutes at 37° C and at a pH of about 6.8. A buffer solution can be used to perform this test, for example a 0.05 M sodium phosphate buffer solution at pH 6.8.

The film thickness appeared to not be of significant influence of the dissolution rate. Therefore, the dissolution tests can be done with films as normally suitable for - for example - hard capsules or soft capsules.

Preferably, if it would be necessary or useful, the dissolution test for films suitable as soft capsules would be performed on 1 mm films, while preferably the tests on films suitable for hard capsules would be performed on films of 0.1 mm.

More preferably, the capsule comprising the film shows enteric behavior, as defined according to the United States Pharmacopeia (USP). The USP requires an acid stage for a duration of 1 hour, wherein the medium comprises 0.084 M HCI, 0.034 M NaCl and 0.32% w/v pepsin. During this stage, less than 10% of the active ingredient may be present in the solution. It further requires a pH 6.8 buffer stage: the medium comprises 0.05 M phosphate buffer, 0.05 M K⁺, 0.022 M Na⁺, gastric enzymes, and has an ionic strength of 0.094 M. After 45 minutes, more than 85% of the active ingredient should be present in the solution.

More preferably, the capsule comprising the film shows enteric behavior, as defined according to the European Pharmacopoeia (Ph. Eur.). The Ph.Eur. requires an acid stage for 2 hours with a 0.1 M HCI medium, during which the active ingredient should not be present in the solution. The pH 6.8 Buffer stage comprises 0.154 M buffer with an ionic strength of 0.463 M. The counterion is Na⁺. After 45 minutes, more than 85% of the active ingredient should be present in the solution.

Even more preferably, the capsule comprising the film passes the disintegration tests defined in the European Pharmacopoeia. This means that during the acid stage, no defects should be observed in the capsules. The film should disintegrate during the second stage.

In one preferred embodiment, the film forms a hard capsule shell comprising a capsule cap and a capsule body. Hard capsules are preferred for administering solid products. The hard capsule shell preferably comprises type B gelatin (alkali processed), but may comprise type A (acid treated) gelatin.

Preferably, the hard capsule shell comprises gelatin of the "high Bloom" type, and more preferably with a Bloom strength of about 200 to about 280 g Bloom. The gelatin preferably has a viscosity of about 4 to about 5 mPa s.

Preferably, the film forms a soft capsule shell. Soft gelatin capsules generally contain type B gelatin (alkali processed), but may comprise type A (acid treated) gelatin. The gelatin preferably is of the "medium Bloom" type, and more preferably has a Bloom strength of about 120 to about 220 g Bloom. The gelatin preferably has a viscosity of about 2 to about 4 mP s.

The thickness of the film may be between 0.02 to 2 mm, and preferably has a thickness of between 0.05 to 1.5 mm.

Generally, hard capsules comprise a gelatin film of between 0.05 to 0.15 mm, while soft capsules have a film thickness between 0.5 to 1.2 mm. Films for other purposes generally will have a thickness in this range. Such thicknesses are particularly preferred for the film according to the present invention.

The invention further provides a capsule, wherein the film of the invention encapsulates a filling material, generally an active ingredient. The filling material comprises an active ingredient like, preferably, a food supplement or an active pharmaceutical ingredient.

In one preferred embodiment, the capsule is a hard capsule. The hard capsule may comprise liquid ingredients or solid ingredients, preferably it comprises solid ingredients such as powders, pellets, granules or mini tablets.

In another preferred embodiment, the capsule is a soft capsule. The soft capsule preferably comprises liquids, oils, pastes, emulsions or suspensions.

### Process

The present invention provides a method of preparing a film. The method comprises the steps of: (a) preparing an aqueous gelatin solution comprising 20 wt.% or more and 50% or lower of gelatin and an effective amount of surfactant to achieve a slow dissolution rate at low pH while having a fast dissolution rate at neutral pH, and (b) forming a film.

In a particularly preferred embodiment, the solution comprises 6 wt.% or more of an anionic surfactant having a C₈-C₂₄ alkyl chain.

The aqueous gelatin solution is prepared by dissolving the gelatin and the anionic surfactant in water.

Preferably, the water is deionized water.

Preferably, the aqueous gelatin solution has a pH of about 3 to about 6.

In case soft gelatin capsules are prepared, a plasticizer is added, preferably in an amount of about 10 to about 25 wt% of the solution.

In one embodiment, an acid is added. In case an acid is used, the acid is citric acid or lactic acid, more preferably it is lactic acid. The acid can among others allow the aqueous gelatin solution to reach the desired pH.

Preferably, dissolving the gelatin and the anionic surfactant is effected under constant stirring.

Preferably, dissolving the gelatin and the anionic surfactant is effected at a temperature of about 50 °C to about 75 °C.

Preferably, the dissolution and heating is effected until the gelatin is dissolved. More preferably, the dissolution and heating is effected for about 10 minutes to about 30 minutes.

Preferably, the solution is filtered. This removes solid contaminants, which can cause faults in the film. More preferably, the solution is filtered through a mesh of from 6 to 20 mesh (3.3 mm to 0.8 mm screen).

Preferably, the solution is degassed under reduced pressure. Degassing reduces incidence of bubbles, which can result in faults in the film.

Preferably, step (b) comprises (i) forming the film into a capsule cap and/or a capsule body, and (ii) drying said capsule cap and/or capsule body to yield the capsule body or capsule cap of the invention.

The forming of a capsule cap and/or a capsule body may be performed according to any method known in the art. Generally, this is performed by dipping pairs (body and cap) of standardized steel pins arranged in rows on metal bars into the aqueous gelatin solution. Following adsorption of the gelatin solution on to the surface of the pins, the bar containing the pins is removed and rotated several times to evenly distribute the solution around the pins and to form the capsule cap and/or capsule body. Said capsule cap and capsule body may then be passed through several drying stages to achieve the desired moisture content and yield the capsule bodies or capsule caps of the invention.

In one preferred embodiment of the present invention, the method in step (b) comprises:
(i) forming the film into a capsule cap and/or a capsule body, and
(ii) drying said capsule cap and/or capsule body

In another preferred embodiment of the present invention, the method in step (b) comprises
(i) encapsulating filling material comprising a food supplement or an active pharmaceutical ingredient within the film to form a soft capsule; and
(ii) drying said soft capsule.

The encapsulation process can be performed using methods known in the art, such as: plate process, rotary die process, reciprocating die process, accogel process, seamless process, and the like.

The invention further relates to a use of an effective amount of surfactant in a gelatin based film to provide a slow dissolution rate at low pH, in comparison to a fast dissolution rate at neutral pH.

Preferably the amount of surfactant is such to provide a dissolution rate such that 50% or less of a 1 mm film is dissolved within 120 minutes at 37° C at a pH of about 1, while the dissolution rate at pH 6.8 is more than 50% in 45 min or less.

The invention further relates to a use of an effective amount of surfactant in a gelatin based film to provide a capsule having delayed release behavior and preferably enteric behavior.

### EXAMPLES

For dissolution tests, the following amounts were used:

| Type of film | Weight per fragment | Thickness per fragment |
|---|---|---|
| Soft Capsule | 0.5 + / - 0.05g | 0.5 - 1.2 mm |
| Hard Capsule | 75 +/- 1 mg | 0.085 - 0.12 mm |

### Examples 1-2, comparative examples A-B

### Gelatin film preparation

The gelatin used in the examples is an acid treated bone gelatin. It has a Bloom strength of 180 g Bloom, and a viscosity of 2.5 mPa s. Measurements were performed in line with the GME version number 15 of October 2020.

Gelatin, glycerol and sodium lauryl sulfate were dissolved in deionized water according to Table 1, while heated to 70 °C during 2 hours in an ultrasonic bath.

Glycerol is used as 86% solution in water. The amounts on dry basis are calculated on 100% glycerol.

**Table 1: Gelatin solution composition and composition of the film.**

| | **A** | | **B** | | **1** | | **2** | |
|---|---|---|---|---|---|---|---|---|
| | Wet basis | Dry basis | Wet basis | Dry basis | Wet basis | Dry basis | Wet basis | Dry basis |
| DI water | 40 | -12 | 35 | -12 | 36.5 | -12 | 30 | -12 |
| SLS | - | - | 5 | 7.1 | 8 | 11.6 | 10 | 13.1 |
| Glycerol (86%) | 20 | 26.5 | 20 | 24.3 | 18.5 | 23,0 | 20 | 22.5 |
| Gelatin | 40 | 61.5 | 40 | 56.6 | 37 | 53,5 | 40 | 52.4 |

The resulting mixtures were cooled to room temperature (22°C), formed into films and left to solidify. The gelatin films were dried to reach a moisture of 12%. The thickness dimensions of the prepared films are presented below.

**Table 2: thickness of gelatin films.**

| | **A** | **B** | **1** | **2** |
|---|---|---|---|---|
| Thickness (mm) | 0.9-1.1 | 0.7-0.8 | 0.5-0.7 | 0.6-0.7 |

### Dissolution test

The obtained gelatin films were evaluated for their delayed dissolution properties. Dissolution tests were performed by monitoring the amount of gelatin film dissolved as a function of time. Gelatin films were inserted in a sinker, placed in a beaker comprising a stirring rod and 750 ml of a 0.1M HCI solution (pH 1) for 120 minutes. Thereafter, 250 ml of a 0.2 M Na₃PO₄ solution was added, and the pH was adjusted to 6.8. The beaker was stirred for a further 45 minutes. The amount of gelatin in solution was determined spectrophotometrically at 218 nm over time.

The results are shown in Table 3.

**Table 3: Delayed dissolution properties of gelatin films.**

| Phase | Time (min) | **A** | **B** | **1** | **2** |
|---|---|---|---|---|---|
| | | Diss (%) | Diss (%) | Diss (%) | Diss (%) |
| Acid phase | 0 | 0 | 0 | 0 | 0 |
| | 30 | 98 | 95 | 8 | 7 |
| | 60 | 100 | 100 | 13 | 11 |
| | 90 | 99 | 99 | 19 | 14 |
| | 120 | 101 | 101 | 25 | 18 |
| Phosphate phase | 120 | 101 | 101 | 38 | 42 |
| | 135 | 100 | 100 | 68 | 75 |
| | 165 | 100 | 100 | 100 | 100 |

Example gelatin films 1 and 2 were found almost intact after 120 minutes in the acid medium. After exposure to the buffer medium, dissolution of samples 1 and 2 was rapid and complete. Samples 1 and 2 therefore will showed the desired delayed release when used as a capsule film.

### Example 3

An experiment with 6 wt.% SDS (9 wt.% in the dried film) showed clear delayed dissolution in acid with 50% dissolution in 30 min, and 65% dissolution in 60 min.

### Examples 4-6, Comparative Example A

Gelatin films according to Table 4 were prepared using the same method, and assessed using the same method as in Example 1.

**Table 4: Gelatin solution composition and composition of the films.**

| | **A** | | **4** | | **5** | | **6** | |
|---|---|---|---|---|---|---|---|---|
| | Wet basis | Dry basis | Wet basis | Dry basis | Wet basis | Dry basis | Wet basis | Dry basis |
| DI water | 40 | ∼12 | 28.5 | ∼12 | 27.5 | ∼12 | 27.5 | ∼12 |
| SLS | - | - | 8 | 10.2 | 10 | 12.6 | 10 | 12.6 |
| Lactic acid | - | - | 8 | 10.2 | 10 | 12.6 | - | |
| Citric acid | - | - | - | - | - | - | 10 | 12.6 |
| Glycerol (86%) | 20 | 26.5 | 18.5 | 20.3 | 17.5 | 19.0 | 17.5 | 19.0 |
| Gelatin | 40 | 61.5 | 37 | 47.3 | 35 | 43.9 | 35 | 43.9 |

The thickness dimensions of the prepared films are presented below in Table 5.

**Table 5: thickness of films.**

| | **A** | **4** | **5** | **6** |
|---|---|---|---|---|
| Thickness (mm) | 0.9-1.1 | 1.0 - 1.3 | 0.7-1.1 | 0.8 - 1.0 |

The results of the examination on delayed dissolution properties are shown in Table 6.

**Table 6: Delayed dissolution properties of gelatin films.**

| Phase | Time (min) | **A** | **4** | **5** | **6** |
|---|---|---|---|---|---|
| | | Diss (%) | Diss (%) | Diss (%) | Diss (%) |
| Acid phase | 0 | 0 | 7 | 0 | 0 |
| | 30 | 98 | 15 | 7 | 8 |
| | 60 | 100 | 22 | 11 | 13 |
| | 90 | 99 | 27 | 15 | 16 |
| | 120 | 101 | 31 | 18 | 19 |
| Phosphate phase | 120 | 101 | 63 | 49 | 61 |
| | 135 | 100 | 92 | 86 | 90 |
| | 165 | 100 | 99 | 100 | 100 |

Example gelatin films 5 and 6 were found almost intact after 120 minutes in the acid medium. After exposure to the buffer medium, dissolution of Samples 5 and 6 was rapid and complete and thus fulfilling the requirements for a delayed release capsule. Gelatin film A was readily dissolved and thus will exhibit no delayed release properties. Gelatin film 4 showed delayed dissolution, even though maybe not sufficient for full enteric behavior, such film could be used for capsules showing delayed, gradual release in the stomach and intestines.

### Examples 7-9, Comparative Example C

Hard capsule films were prepared using gelatin Bloom 243 having a viscosity of 4.6 mPa s following the process of Example 1 in according with Table 7.

**Table 7: Gelatin solution composition and composition of the films**

| | **C** | | **7** | | **8** | | **9** | |
|---|---|---|---|---|---|---|---|---|
| | Wet basis | Dry basis | Wet basis | Dry basis | Wet basis | Dry basis | Wet basis | Dry basis |
| DI water | 70 | 12 | 67 | 12 | 64 | 12 | 62 | 12 |
| SLS | - | - | 3 | 8 | 6 | 14,7 | 8 | 18,5 |
| Gelatin | 30 | 88 | 30 | 80 | 30 | 74,3 | 30 | 69,5 |

Films were prepared with a thickness of about 0.1 mm. The results of the examination on delayed dissolution properties are shown in Table 8.

**Table 8: Delayed dissolution properties of gelatin films.**

| Phase | Time (min) | **C** | **7** | **8** | **9** |
|---|---|---|---|---|---|
| | | Diss (%) | Diss (%) | Diss (%) | Diss (%) |
| Acid phase | 0 | 0 | 0 | 0 | 0 |
| | 30 | 83 | 76 | 32 | 7 |
| | 60 | 97 | 95 | 45 | 13 |
| | 90 | 100 | 98 | 51 | 18 |
| | 120 | 100 | 99 | 56 | 23 |
| Phosphate phase | 120 | 100 | 100 | 85 | 70 |
| | 135 | 100 | 100 | 90 | 90 |
| | 165 | 100 | 100 | 94 | 100 |

Example gelatin film 9 was found almost intact after 120 minutes in the acid medium. After exposure to the buffer medium, dissolution of Sample 9 was rapid and complete and thus fulfilling the requirements for use as a delayed release capsule. Gelatin films C was readily dissolved and thus will exhibit no delayed release properties. Gelatin film 7 showed a slight retarded dissolution, and a capsule comprising said film may exhibit a slight delayed release. Gelatin film 8 will show a clear delayed release, even though maybe not sufficient for full enteric behavior of a capsule. Films 7 and 8 could be used for delayed, gradual release in the stomach and intestines.

These experiments show that the presence of a substantial amount of surfactant is also effective in hard capsule films to provide delayed dissolution of the film.

## Claims

1. A homogeneous solid composition comprising gelatin and a surfactant, wherein the dissolution rate of the solid composition in aqueous medium at pH 6.8 is higher than the dissolution rate in aqueous medium at pH 1.

2. The composition of claim 1 **characterized in that** the composition is a film.

3. The composition or film according to any one of claims 1-2, wherein the dissolution rate is such that
(i) 50% or less of the composition or film is dissolved within 120 minutes at 37 °C at a pH of about 1, and
(ii) 50% or more of the composition or film is dissolved within 45 minutes or less at pH 6.8 at 37°C.

4. The composition or film according to any one of claims 1-3, wherein the surfactants include anionic surfactants, cationic surfactants, non-ionic surfactants and amphoteric surfactants.

5. The composition or film according to any one of claims 1-4, wherein the composition or film comprises gelatin in an amount of about 30 wt.% or higher, and 80 wt.% or lower and about 8 wt.% or more on dry weight basis of an anionic surfactant having a C₈-C₂₄ alkyl chain, preferably about 10 wt % or more.

6. The composition or film according to any one of the preceding claims, wherein the anionic surfactant comprises an anionic group and an alkyl-chain moiety, wherein the anionic group is a sulfate group, sulfonic group, phosphate group, phosphonate group, or carboxylate group, and preferably wherein the anionic group is a strong acid group like a sulfate group or sulfonic acid group.

7. The composition or film according to any one of the preceding claims, wherein the surfactant comprises ether or ester groups.

8. The composition or film according to any one of the preceding claims, wherein the molecular weight of the surfactant is about 250 g/mol to about 600 g/mol, preferably about 300 g/mol to about 400 g/mol.

9. The composition or film according to any of the preceding claims, wherein the anionic surfactant is chosen from the group consisting of sodium lauryl sulfate (SLS), sodium lauryl ether sulfate, ammonium lauryl sulfate, ammonium lauryl ether sulfate, sodium stearate, magnesium stearate, calcium stearate, sodium stearyl fumarate, calcium stearyl fumarate, sodium stearoyl-2-lactylate, calcium stearoyl-2-lactylate, dioctyl sodium sulfosuccinate, sodium cetearyl sulfate; preferably chosen from the group consisting of sodium lauryl sulfate (SLS) and sodium lauryl ether sulfate; and more preferably sodium lauryl sulfate (SLS).

10. The composition or film according to any of the preceding claims, wherein the amount of gelatin is 35 wt.% or higher, and 80 wt.% or lower on dry weight basis.

11. The composition or film according to any of the preceding claims, wherein the amount of the surfactant is 8 wt.% or higher, preferably 10 wt.% or higher, and 30 wt.% or lower, preferably 20 wt.% or lower.

12. The composition or film according to any of the preceding claims, wherein the composition or film further comprises at least one plasticizer selected from the group consisting of glycerol, propylene glycol, polyethylene glycol, sugar alcohols with 3 to 6 carbon atoms, organic esters such as triethyl citrate and diethyl phthalate, and combinations thereof, preferably wherein said plasticizer is selected from the group consisting of glycerol, sorbitol, propylene glycol, or combinations thereof, more preferably wherein said plasticizer comprises glycerol.

13. The composition or film according to any of the preceding claims, wherein the composition or film comprises at least one plasticizer wherein the weight of plasticizer is about 10 to about 40 wt.% on dry weight basis, preferably about 15 wt.% to about 30 wt.%.

14. A hard capsule comprising a capsule shell, wherein the capsule shell comprises, preferably consists of the film according to claims 2 to 11.

15. A soft capsule comprising a capsule shell, wherein the capsule shell comprises, preferably consists of the film according to claims 2 to 13.

16. A capsule according to any of claims 14-15, wherein the film according to any of claims 2-13 encapsulates filling material comprising a food supplement or an active pharmaceutical ingredient.

17. A capsule according to any one of claims 14-16, wherein the film according to any of claims 2-13 is the outer layer of the capsule.
